(19)

Europäisches Patentamt

European Patent Office

Office européen des brevets

(11) **EP 3 632 331 A1**

(12) **EUROPEAN PATENT APPLICATION**

(43) Date of publication:
08.04.2020 Bulletin 2020/15

(51) Int Cl.:
*A61B 8/08* (2006.01)

(21) Application number: 18198791.8

(22) Date of filing: 05.10.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• MEGENS, Mischa
5656 AE Eindhoven (NL)
• STAPERT, Hendrik Roelof
5656 AE Eindhoven (NL)
• GOKGURLER, Mustafa Hakan
5656 AE Eindhoven (NL)

• VAN DE PAS, Stefan
5656 AE Eindhoven (NL)
• KORTSMIT, Jeroen
5656 AE Eindhoven (NL)
• VAN HEESCH, Franciscus Hendrikus
5656 AE Eindhoven (NL)
• BELT, Harm Jan Willem
5656 AE Eindhoven (NL)
• JAIN, Ameet Kumar
5656 AE Eindhoven (NL)
• POLAND, McKee Dunn
5656 AE Eindhoven (NL)
• VAIDYA, Kunal
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **INTERVENTIONAL DEVICE POSITIONING USING ULTRASOUND SIGNALS**

(57) A system for determining a position of an interventional device (11) respective an imaging field ($B_{1..k}$) corresponding to a type ($T_{1..n}$) of a beamforming ultrasound imaging probe (13) currently connected to an ultrasound imaging system (14). The position is determined based on ultrasound signals transmitted between the beamforming ultrasound imaging probe (13) and an ultrasound transducer (15) attached to the interventional device (11). An image reconstruction unit (IRU) provides a reconstructed ultrasound image (RUI) corresponding to the imaging field ($B_{1..k}$). A position determination unit (PDU) receives input indicative of the type ($T_{1..n}$) of the beamforming ultrasound imaging probe (13) currently connected to the ultrasound imaging system (14). The position determination unit (PDU) also computes a position ($LAP_{TOFSmax, \theta IPA}$) of the ultrasound transducer (15) respective the imaging field ($B_{1..k}$). Computing the position ($LAP_{TOFSmax, \theta IPA}$) comprises selecting from a group of beam sequences corresponding to a plurality of imaging probe types ($T_{1..n}$) a beam sequence corresponding to the type ($T_{1..n}$) of the beamforming ultrasound imaging probe (13) currently connected to the ultrasound imaging system (14) and assigning detected ultrasound signals to the selected beam sequence.

FIG. 1

**Description**

FIELD OF THE INVENTION

The invention relates to determining a position of an interventional device respective an imaging field of a beamforming ultrasound imaging probe.

BACKGROUND OF THE INVENTION

Interventional devices such as medical needles, catheters and surgical tools are often difficult to visualize in an ultrasound image due to the specular nature of their reflectivity, particularly at unfavorable incidence angles.

In this respect documents WO2011138698A1, WO2015101949A1 and WO2016009350A1 describe systems for tracking an instrument in an ultrasound imaging field with an ultrasound receiver that is mounted to the instrument. The position of the ultrasound receiver is subsequently displayed in an ultrasound image corresponding to the ultrasound imaging field.

In planar ultrasound imaging systems, determining a position of such an ultrasound receiver respective a plane becomes particularly challenging when the ultrasound receiver lies outside the image plane, i.e. is "out-of-plane".

In this respect, document WO2018060499A1 describes a system for indicating a position of an interventional device feature of an interventional device respective an image plane defined by an ultrasound imaging probe of a beamforming ultrasound imaging system in which the position of the interventional device feature is determined based on ultrasound signals transmitted between the ultrasound imaging probe and an ultrasound transducer attached to the interventional device at a predetermined distance from the interventional device feature. An icon providing unit provides a first icon indicative of a circular zone with a radius corresponding to the predetermined distance. The first icon is displayed in a fused image that includes a reconstructed ultrasound image from the beamforming ultrasound imaging system. In this document an out-of-plane distance is computed based on a model of the variation in signal intensity with out-of-plane distance $D_{op}$ for the determined range.

Despite these solutions there remains room for improved techniques for determining a position of an interventional device respective an ultrasound imaging field.

SUMMARY OF THE INVENTION

In seeking to provide improved tracking of an interventional device, a system is provided for determining a position of an interventional device respective an imaging field corresponding to a type of a beamforming ultrasound imaging probe currently connected to an ultrasound imaging system and in which the position of the interventional device is determined based on ultrasound signals transmitted between the beamforming ultrasound imaging probe and an ultrasound transducer attached to the interventional device. The system includes an image reconstruction unit and a position determination unit. The image reconstruction unit provides a reconstructed ultrasound image corresponding to the imaging field defined by the beamforming ultrasound imaging probe. The position determination unit receives input indicative of the type of the beamforming ultrasound imaging probe currently connected to the ultrasound imaging system. The position determination unit also computes a position of the ultrasound transducer respective the imaging field based on a time of flight of a maximum detected intensity ultrasound signal transmitted between the beamforming ultrasound imaging probe and the ultrasound transducer. Computing the position comprises selecting from a group of beam sequences corresponding to a plurality of imaging probe types a beam sequence corresponding to the type of the beamforming ultrasound imaging probe currently connected to the ultrasound imaging system and assigning detected ultrasound signals to the selected beam sequence. The position determination unit also indicates the position in the reconstructed ultrasound image.

In the invention a position of the interventional device is determined based on ultrasound signals transmitted between the beamforming ultrasound imaging probe and the ultrasound transducer attached to the interventional device. More specifically, the beam in which the transmitted ultrasound signals are detected with maximum intensity, i.e. the "maximum detected intensity ultrasound signal transmitted between the beamforming ultrasound imaging probe and the ultrasound transducer" identifies the beam in which the ultrasound transducer is located, and consequently the angular position of the transducer respective the ultrasound imaging probe.

Beamforming ultrasound imaging probes are typically identified by a type. The type may be defined at a general level such as curved or linear, and in more detail may include the name of the manufacturer, and ultimately details such as a model number and so forth. Typically the ultrasound imaging field, i.e. a region within which ultrasound signals are transmitted and detected by the probe in order to provide a reconstructed ultrasound image, is specific to the probe type. Moreover, information describing the beam sequence of the probe, i.e. the temporal order in which each of its beams are transmitted, may be used by the position determination unit in order to temporally match the detected ultrasound signals with those that were transmitted, and consequently determine, via the maximum detected intensity ultrasound signal, in which beam the ultrasound transducer is located.

In the invention a beam sequence corresponding to the type of the beamforming ultrasound imaging probe currently connected to the ultrasound imaging system, is selected from a group of beam sequences. Each beam sequence in the group corresponds to a specific imaging probe type. The detected ultrasound signals are then assigned to the selected beam sequence. Obtaining the beam sequence for the ultrasound imaging probe currently connected to the ultrasound imaging system in this manner means that detected ultrasound signals can be reliably associated with the correct transmitted beam. Moreover it allows the position determination unit to operate with multiple different probe types. In some implementations the intensity of each detected ultrasound signal may optionally be mapped into a common multidimensional array, this being subsequently analyzed by a common algorithm to determine the maximum intensity. Providing a common data structure for all probe types that may be analyzed by a common algorithm, e.g. to determine the maximum detected intensity ultrasound signal, alleviates the need for different algorithms for each probe type. The common algorithm also provides consistency in the results for different probe types. Consequently the maximum detected intensity ultrasound signal transmitted between the beamforming ultrasound imaging probe and the ultrasound transducer can be reliably determined for multiple types of ultrasound imaging probes.

In accordance with one aspect the imaging field of the ultrasound imaging probe comprises an image plane, and computing the position of the ultrasound transducer respective the imaging field includes determining an out-of-plane distance between the ultrasound transducer and the image plane. The out-of-plane distance is determined based on the intensity and the time of flight of the maximum detected intensity ultrasound signal. Moreover, determining the out-of-plane distance includes selecting from a group of models, a model corresponding to the type of the beamforming ultrasound imaging probe currently connected to the ultrasound imaging system. The model describes an expected variation of in-plane maximum detected intensity with time of flight. Determining the out-of-plane distance also includes comparing the maximum detected intensity with the selected model, at the time of flight of the maximum detected intensity ultrasound signal. Indicating the position in the reconstructed ultrasound image further includes indicating the out-of-plane distance in the reconstructed ultrasound image.

When the imaging field is a plane, a user is often interested in determining the position of the interventional device respective the image plane. By virtue of the finite thickness of the ultrasound imaging plane, the ultrasound imaging field extends a short distance either side of the image plane, i.e. for a short out-of-plane distance. In this aspect of the invention the ultrasound signals in this out-of-plane region can be used to determine the position of the interventional device. The position determination unit computes a position of the ultrasound transducer respective the imaging field in the same manner as described above, i.e. based on the ultrasound signals transmitted between the ultrasound imaging probe and the ultrasound transducer, this position now being a lateral position respective the image plane. Moreover, the out-of-plane distance is also indicated in the reconstructed ultrasound image. The model used in determining the out of plane distance describes an expected variation of in-plane maximum detected intensity with time of flight. This variation has been found to be repeatable for imaging probes of the same type, and advantageously only models the variation in one dimension, i.e. time of flight. The out-of-plane distance is determined by comparing, e.g. scaling, the maximum detected intensity with the selected model, at the time of flight of the maximum detected intensity ultrasound signal. This provides a qualitative indication of the out-of-plane distance. Moreover, in-use the out-of-plane distance can be provided quickly since a lookup in only one, i.e. time of flight, dimension is required. By selecting the appropriate model from a group of models it is provided that the system can operate reliably with different types of ultrasound imaging probe.

In accordance with another aspect the position determination unit indicates the position only if the type of the probe currently connected to the beamforming ultrasound imaging system corresponds to a type of probe in a group of two or more supported probe types. Consequently, whilst the ultrasound imaging system may operate with a variety of different probe types it is prevented that an incorrect position is displayed for probe types that are not supported by the position determination unit.

In accordance with other aspects a method and corresponding computer program product that may be used in conjunction with the system are provided.

It is to be noted that the various aspects described in relation to the system may be combined to provide further advantageous effects. Moreover, aspects of the system may be used interchangeably with the method, and vice versa.

BRIEF DESCRIPTION OF THE FIGURES

Fig. 1 illustrates a beamforming ultrasound imaging system 14 in combination with an interventional device 11 having an in-plane ultrasound transducer 15 and an embodiment of the invention in the form of system 10

Fig. 2 illustrates a reconstructed ultrasound image RUI in which a computed position $LAP_{TOFSmax, \theta IPA}$ of the inter-

ventional device is indicated via circle $C_1$.

Fig. 3 illustrates a beamforming ultrasound imaging system 14 in combination with an interventional device 11 having an out-of-plane ultrasound transducer 15 disposed at an out-of-plane distance $D_{op}$ and an embodiment of the invention in the form of system 10.

Fig. 4 illustrates a model $MO_1$ describing an expected variation of in-plane maximum detected intensity, $I_{SmaxInplane}$ (dB) with time of flight, TOF.

Fig. 5A, Fig. 5B, Fig. 5C each illustrate a reconstructed ultrasound image RUI that includes region of interest ROI and an icon $C_{op}$ that is indicative of a circular zone with a radius corresponding to out-of-plane distance $D_{op}$.

Fig. 6 illustrates an interventional device 11 that is suitable for use with system 10.

Fig. 7 illustrates various method steps of a method that may be used with system 10.

## DETAILED DESCRIPTION OF THE INVENTION

In order to illustrate the principles of the present invention, various systems are described in which the position of an interventional device, exemplified by a medical needle, is indicated respective an image plane defined by a linear array of a 2D beamforming ultrasound imaging probe. Moreover, in some examples the position of a feature of the medical device is also tracked. The feature may be its distal end, for example.

It is however to be appreciated that the invention also finds application with other interventional devices such as, and without limitation, a catheter, a guidewire, a probe, an endoscope, an electrode, a robot, a filter device, a balloon device, a stent, a mitral clip, a left atrial appendage closure device, an aortic valve, a pacemaker, an intravenous line, a drainage line, a surgical tool, a tissue sealing device, a tissue cutting device or an implantable device. The tracked feature of such interventional devices may exemplarily include a distal end of the interventional device, a biopsy sampling point of the interventional device, a cutting edge of the interventional device, an opening of a channel in the interventional device, a sensor (e.g. for sensing flow, pressure, temperature etc.) of the interventional device, a surgical tool (e.g. a scraper) integrated in the interventional device, a drug delivery point of the interventional device, or an energy delivery point of the interventional device.

Furthermore it is to be appreciated that the exemplified linear array of a 2D beamforming ultrasound imaging probe is only one example of an ultrasound transceiver array of a beamforming ultrasound imaging system in which the invention may be used. The invention also finds application in other types of beamforming ultrasound imaging systems whose associated ultrasound transceiver arrays exemplarily include a 2D array of a 3D imaging probe (or in bi-plane view), a "TRUS" transrectal ultrasonography probe, an "IVUS" intravascular ultrasound probe, a "TEE" transesophageal probe, a "TTE" transthoracic probe, a "TNE" transnasal probe, an "ICE" intracardiac probe.

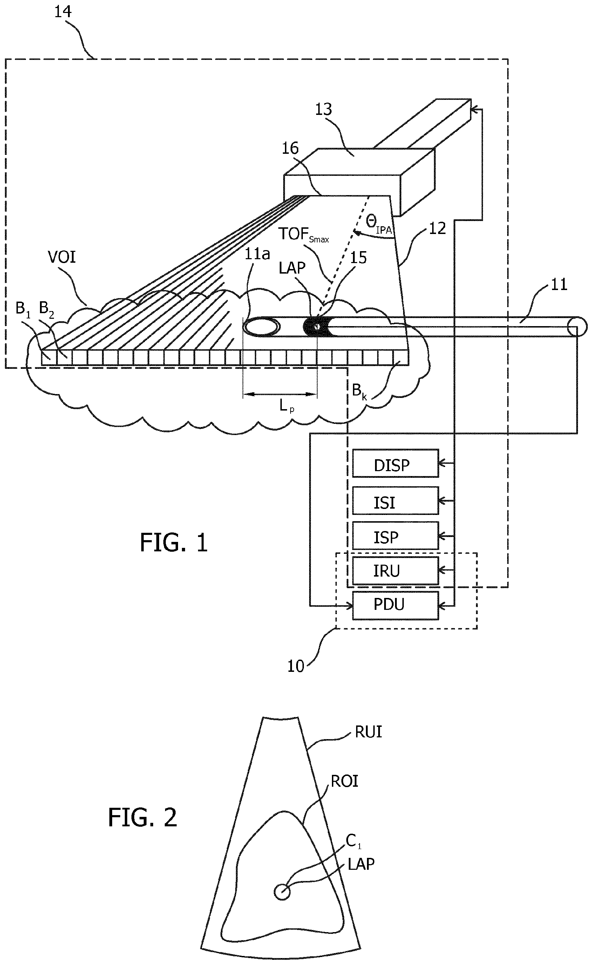

Fig. 1 illustrates a beamforming ultrasound imaging system 14 in combination with an interventional device 11 having an in-plane ultrasound transducer 15 and an embodiment of the invention in the form of system 10. In Fig. 1, beamforming ultrasound imaging system 14 includes a 2D beamforming ultrasound imaging probe 13 which is in communication with image reconstruction unit IRU, imaging system processor ISP, imaging system interface ISI and display DISP. The units IRU, ISP, ISI and DISP are conventionally located in a console that is in wired communication with 2D beamforming ultrasound imaging probe 13. It is also contemplated that wireless communication, for example using an optical, infrared, or an RF communication link, may replace the wired link. It is also contemplated that some of units IRU, ISP, ISI and DISP may instead be incorporated within 2D beamforming ultrasound imaging probe 13, as in for example the Philips Lumify ultrasound imaging system. In Fig. 1, 2D beamforming ultrasound imaging probe 13 includes linear ultrasound transceiver array 16 that transmits and receives ultrasound energy within an ultrasound field that intercepts volume of interest VOI. The ultrasound field is fan-shaped in Fig. 1 and includes multiple ultrasound beams $B_{1..k}$ that define image plane 12. Note that a fan-shaped beam is illustrated in Fig. 1 for the purposes of illustration only and that the invention is not limited to a particular shape of ultrasound field. Beamforming ultrasound imaging system 14 may also include electronic driver and receiver circuitry, not shown, that is configured to amplify and/ or to adjust the phase of signals transmitted by or received by 2D beamforming ultrasound imaging probe 13 in order to generate and detect ultrasound signals in beams $B_{1..k}$. The electronic driver and receiver circuitry may thus be used to steer the emitted and/ or received ultrasound beam direction.

In-use, beamforming ultrasound imaging system 14 is operated in the following way. An operator may plan an ultrasound procedure via imaging system interface ISI. Once an operating procedure is selected, imaging system interface ISI triggers imaging system processor ISP to execute application-specific programs that generate and interpret the signals transmitted by and detected by 2D beamforming ultrasound imaging probe 13. Beamforming ultrasound imaging system 14 may also include a memory (not shown) for storing such programs. The memory may for example store ultrasound beam control software that is configured to control the sequence of ultrasound signals transmitted by and/ or received by beamforming ultrasound imaging probe 13. Image reconstruction unit IRU, which may alternatively form part of imaging system processor ISP, reconstructs data received from the beamforming ultrasound imaging probe 13 into an image corresponding to imaging field $B_{1..k}$, i.e. image plane 12 and which thus intercepts volume of interest VOI, and subsequently displays this image on display DISP. A planar section through volume of interest VOI is termed region of interest ROI herein. Reconstructed ultrasound image RUI may thus include region of interest ROI. The reconstructed image may for example be an ultrasound Brightness-mode "B-mode" image, otherwise known as a "2D mode" image, a "C-mode" image or a Doppler mode image, or indeed any ultrasound planar image.

Also shown in Fig. 1 is a medical needle 11 as an example of an interventional device, and an embodiment of the invention, system 10, that may be used to indicate a position of interventional device 11, i.e. the medical needle, or more specifically ultrasound transducer 15 attached thereto, respective imaging field $B_{1..k}$, i.e. image plane 12 of beamforming ultrasound imaging probe 13. This embodiment, system 10, includes image reconstruction unit IRU and position determination unit PDU. These units are in communication with one another as illustrated by the interconnecting arrows. It is also contemplated that one or more of units PDU, IRU may be incorporated within a memory or a processor of beamforming ultrasound imaging system 14, for example within a memory or a processor that also provides the functionality of unit ISP. Medical needle 11 that is tracked, includes ultrasound transducer 15 that may be positioned at predetermined distance $L_p$ from distal end 11a of interventional device 11.

In-use, a position of interventional device 11, or more specifically that of ultrasound transducer 15 attached thereto, is computed respective imaging field $B_{1..k}$, i.e. image plane 12 by position determination unit PDU based on ultrasound signals transmitted between ultrasound transceiver array 16 and ultrasound transducer 15. The computed position may subsequently be indicated in reconstructed ultrasound image RUI, as seen in Fig. 2, which illustrates a reconstructed ultrasound image RUI in which a computed position $LAP_{TOFSmax, \theta IPA}$ of the interventional device is indicated via circle $C_1$.

In one configuration ultrasound transducer 15 is a detector that receives ultrasound signals corresponding to beams $B_{1..k}$. Position determination unit PDU identifies the lateral position LAP of ultrasound transducer 15 respective imaging field $B_{1..k}$, i.e. image plane 12 by correlating; i.e. comparing, the ultrasound signals emitted by ultrasound transceiver array 16 with the ultrasound signals detected by ultrasound transducer 15. More specifically this correlation determines the best fit position of ultrasound transducer 15 respective imaging field $B_{1..k}$, i.e. image plane 12 based on i) the intensities of the ultrasound signals corresponding to each beam $B_{1..k}$ that are detected by ultrasound transducer 15 and ii) based on the time delay, i.e. time of flight, between emission of each beam $B_{1..k}$ and its detection by ultrasound transducer 15. This may be illustrated as follows. When ultrasound transducer 15 is in the vicinity of imaging field $B_{1..k}$, i.e. image plane 12, ultrasound signals from the nearest of beams $B_{1..k}$ to the transducer will be detected with a relatively larger intensity whereas more distant beams will be detected with relatively smaller intensities. Typically the beam that is detected with the maximum detected intensity is identified as the one that is closest to ultrasound detector 15. In other words, the maximum detected intensity $I_{Smax}$ ultrasound signal identifies the in-plane angle $\Theta_{IPA}$ between ultrasound transceiver array 16 and ultrasound transducer 15. The time of flight, between the emission of this beam (from beams $B_{1..k}$) and its subsequent detection is indicative of the range between ultrasound transceiver array 16 and ultrasound transducer 15. Thus the time delay of the ultrasound signal in the beam that was detected with maximum detected intensity, $I_{Smax}$, i.e. $TOF_{Smax}$, is the ultrasound signal that is selected from the ultrasound signals of all beams. Since the time of flight is indicative of the range, in polar coordinates the lateral position of ultrasound transducer 15 respective imaging field $B_{1..k}$, i.e. image plane 12 maybe represented by $LAP_{TOFSmax, \theta IPA}$. If desired, the range may be determined by multiplying the time delay by the speed of ultrasound propagation.

In another configuration ultrasound transducer 15 is an emitter that emits one or more ultrasound pulses. Such pulses may for example be emitted during tracking frames that are interleaved between the usual imaging frames of ultrasound imaging system 14. In such a tracking frame the ultrasound transceiver array 16 may be operated in a receive-only mode in which it listens for ultrasound signals originating from the vicinity of imaging field $B_{1..k}$, i.e. image plane 12. Ultrasound transceiver array 16 is thus configured as a one-way receive-only beamformer. Position determination unit PDU identifies from which beam of beams $B_{1..k}$ the pulse(s) originated based on the ultrasound signals emitted by ultrasound transducer 15 and those detected by ultrasound transceiver array 16. As in the configuration above, position determination unit PDU may use a correlation procedure that, based on the ultrasound signal detected with maximum intensity and its time of flight, identifies the closest beam and thus the point at which the ultrasound signal was emitted, i.e. its lateral position $LAP_{TOFSmax, \theta IPA}$ in the same manner. Thus, when ultrasound transducer 15 is an emitter, a correlation, i.e. comparison, procedure may again be used to determine its best-fit position respective imaging field $B_{1..k}$, i.e. image plane 12 for each tracking frame.

In another configuration ultrasound transducer 15 may be configured to act as both a receiver and an emitter, or include both a receiver and an emitter. In this configuration ultrasound transducer 15 may be triggered to emit one or more ultrasound pulses upon receipt of an ultrasound signal from ultrasound transceiver array 16; optionally following a delay that is equal to one or more frame periods of ultrasound imaging system 14. In this way the pulse(s) emitted by ultrasound transducer 15 during an imaging mode are received by ultrasound transceiver array 16 in the form of an echo in the reconstructed ultrasound at an angular position, i.e. in an image line, that corresponds to the triggering beam $B_{1..k}$. Ultrasound transducer 15 thus appears as a bright spot in the reconstructed image. Position determination unit PDU may subsequently identify this bright spot in the reconstructed image and thus again compute a position $LAP_{TOFSmax, \theta IPA}$

of ultrasound transducer 15 respective imaging field $B_{1..k}$, i.e. image plane 12.

In yet another configuration, not illustrated, beamforming ultrasound imaging probe 13 may further include at least three ultrasound emitters that are attached to the beamforming ultrasound imaging probe 13. The at least three ultrasound emitters are in communication with position determination unit PDU. Moreover the position determination unit PDU is configured to compute a position of the ultrasound transducer 15 respective the imaging field $B_{1..k}$, i.e. image plane 12 based on ultrasound signals transmitted between the at least three ultrasound emitters attached to the beamforming ultrasound imaging probe 13, and the ultrasound transducer 15. In this configuration position determination unit PDU determines a range between each emitter and ultrasound transducer 15 based on the time of flight of ultrasound signals emitted by each emitter. The three dimensional position of ultrasound transducer 15 is subsequently determined using triangulation. This provides the position of ultrasound transducer 15 in three dimensions respective beamforming ultrasound imaging probe 13, or more specifically respective imaging field $B_{1..k}$, i.e. image plane 12 since the at least three emitters are attached to the beamforming ultrasound imaging probe 13. The three-dimensional position may subsequently be mapped to imaging field $B_{1..k}$, i.e. image plane 12 and thus again represented by $LAP_{TOFSmax, \theta IPA}$. Ultrasound emitters are preferred in this configuration because the supply of high power ultrasound signals to the emitters, necessary for accurate positioning over a large range, is simpler when the emitters are proximate beamforming ultrasound imaging probe 13 where a power source is readily available. This arrangement is thus preferred in contrast to locating a high power emitter on interventional device 11. In-use, the lateral position of interventional device 11, or more specifically that of ultrasound transducer 15 attached thereto, is thus again computed respective imaging field $B_{1..k}$, i.e. image plane 12 by position determination unit PDU based on ultrasound signals transmitted between the at least three emitters and ultrasound transducer 15.

Position determination unit PDU illustrated in Fig. 1 may thus be used in any of the above configurations to compute a position of ultrasound transducer 15 respective imaging field $B_{1..k}$, i.e. image plane 12 based on ultrasound signals transmitted between beamforming ultrasound imaging probe 13 and ultrasound transducer 15. In more general terms, whilst image plane 12 has been used as an example in the above, the same principle may be used to determine a position of ultrasound transducer 15 respective imaging field $B_{1..k}$, i.e. when a volumetric, i.e. three dimensional, imaging field $B_{1..k}$ is provided.

Beamforming ultrasound imaging probes are typically identified by a type, denoted herein as $T_{1..n}$ wherein n is greater than or equal to two. Type $T_{1..n}$ may be defined at a general level such as curved or linear, and in more detail may include the name of the manufacturer, and ultimately a model number. Typically ultrasound imaging field $B_{1..k}$ in Fig. 1, i.e. the region within which ultrasound signals are transmitted and detected by the imaging probe 13 in order to provide reconstructed ultrasound image RUI, is specific to the probe type. Moreover, information describing the beam sequence of the probe, i.e. the temporal order in which each of its beams are transmitted, may be used by position determination unit PDU in order to match the detected ultrasound signals with those that were transmitted, and consequently determine, via the maximum detected intensity ultrasound signal, in which beam the ultrasound transducer is located.

In the invention, position determination unit PDU receives input indicative of the type $T_{1..n}$ of the beamforming ultrasound imaging probe 13 currently connected to the ultrasound imaging system 14 and a beam sequence corresponding to the type $T_{1..n}$ of the beamforming ultrasound imaging probe currently connected to the beamforming ultrasound imaging system is selected from a group of beam sequences. Each beam sequence in the group corresponds to a specific imaging probe type $T_{1..n}$. Parameter n is greater than or equal to two, and thus the group includes at least two beam sequences. Moreover there may be more than one beam sequence associated with each probe type. A beam sequence represents the beams of beamforming ultrasound imaging probe 13 that are generated, and the order in which they are generated. Beam sequences are typically specific to the imaging probe type $T_{1..n}$ in view of the need to image a predetermined field of view, or to provide focusing at a particular region. Thus for example the beam sequence for a curved probe may differ from that for a linear probe. The detected ultrasound signals are then assigned to the selected beam sequence. More specifically, the detected ultrasound signals are assigned to temporally corresponding beams of the selected beam sequence. The input indicative of the type $T_{1..n}$ may be received automatically, for example by position determination unit receiving a code stored in imaging probe 13, or manually, for example via imaging system interface ISI in which a user may select a probe type from a list of two or more supported probe types. The input may alternatively be received via a reader device such as e.g. a barcode reader or an RFID reader that reads a corresponding code stored on imaging probe 13.

The group of beam sequences corresponding to imaging probe types $T_{1..n}$ may exemplarily be stored as a database or a library and thus selected therefrom. This may be provided by a memory. The memory may for example be included within the position determination unit or within another part of ultrasound imaging system 14; for example in a memory of imaging system processor ISP.

In summary, and with reference to Fig. 1 and Fig. 2, a system 10 is provided for determining a position of interventional device 11 respective imaging field $B_{1..k}$ corresponding to a type $T_{1..n}$ of a beamforming ultrasound imaging probe 13 currently connected to an ultrasound imaging system 14 and in which the position of the interventional device

11 is determined based on ultrasound signals transmitted between the beamforming ultrasound imaging probe 13 and an ultrasound transducer 15 attached to the interventional device 11. System 10 includes image reconstruction unit IRU and position determination unit PDU. Image reconstruction unit IRU provides a reconstructed ultrasound image RUI corresponding to imaging field $B_{1..k}$ and which is defined by beamforming ultrasound imaging probe 13. Position determination unit PDU:

> receives input indicative of the type $T_{1..n}$ of the beamforming ultrasound imaging probe 13 currently connected to ultrasound imaging system 14;
> computes a position $LAP_{TOFSmax, \theta IPA}$ of ultrasound transducer 15 respective imaging field $B_{1..k}$ based on a time of flight $TOF_{Smax}$ of a maximum detected intensity $I_{Smax}$ ultrasound signal transmitted between beamforming ultrasound imaging probe 13 and ultrasound transducer 15, wherein computing the position $LAP_{TOFSmax, \theta IPA}$ comprises selecting from a group of beam sequences corresponding to a plurality of imaging probe types $T_{1..n}$ a beam sequence corresponding to the type $T_{1..n}$ of beamforming ultrasound imaging probe currently connected to the ultrasound imaging system 14 and assigning detected ultrasound signals to the selected beam sequence; and
> indicates the position $LAP_{TOFSmax, \theta IPA}$ in reconstructed ultrasound image RUI.

Obtaining the beam sequence for the ultrasound imaging probe currently connected to the ultrasound imaging system and assigning the detected signals thereto in this manner means that detected ultrasound signals can be reliably associated with the correct transmitted beam $B_{1..k}$. Moreover it allows position determination unit PDU to operate with multiple different probe types.

In some implementations the intensity of each detected ultrasound signal may optionally be mapped into a common multidimensional array, this being subsequently analyzed by a common algorithm to determine the maximum intensity. A two-dimensional array may exemplarily have indices of the beam or the image line number and time of flight, and a three-dimensional array may exemplarily have an additional index for the frame number. In the array the beam sequence may be re-ordered from the order in which the beams were detected such that adjacent image lines in the reconstructed ultrasound image are adjacent one another in the array. Image lines as defined herein are adjacent slices in reconstructed ultrasound image RUI in the time of flight, i.e. depth dimension. Providing a common data structure for all probe types that may be analyzed by a common algorithm to determine e.g. the maximum detected intensity ultrasound signal $I_{Smax}$ alleviates the need for different algorithms for each probe type. The common algorithm also provides consistency in the results for different probe types $T_{1..n}$. Consequently the maximum detected intensity ultrasound signal $I_{Smax}$ transmitted between the beamforming ultrasound imaging probe 12 and the ultrasound transducer 15 can be reliably determined for multiple types of ultrasound imaging probes.

In some exemplary implementations, when imaging field $B_{1..k}$ comprises an image plane 12, the type $T_{1..n}$ of the beamforming ultrasound imaging probe may additionally be used in providing a probe-type-specific qualitative indication of the distance of ultrasound transducer 15 from image plane 12, i.e. the out-of-plane distance. The use of the probe type $T_{1..n}$ thus again facilitates operation of the system with different probes.

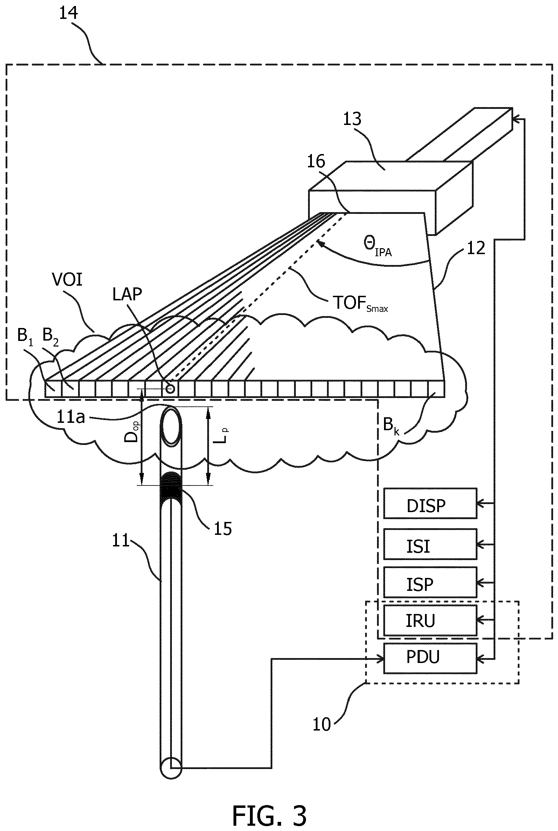

Thereto, Fig. 3 illustrates a beamforming ultrasound imaging system 14 in combination with an interventional device 11 having an out-of-plane ultrasound transducer 15 disposed at an out-of-plane distance $D_{op}$ and an embodiment of the invention in the form of system 10. In contrast to Fig. 1, in Fig. 3 ultrasound transducer 15 is disposed away from the image plane, i.e. is "out-of-plane". With such a planar field it is useful for a user to know the out-of-plane distance in order to allow them to navigate interventional device 11 to image plane 12. In this respect the same procedure as described above may be used to determine a position of ultrasound transducer 15 respective image plane 12, this position now being a lateral position, i.e. a projected position onto image plane 12. An additional procedure that uses the intensity, $I_{Smax}$, and the time of flight, $TOF_{Smax}$, of the maximum detected intensity $I_{Smax}$ ultrasound signal, may subsequently be used to provide a qualitative indication of out-of-plane distance $D_{op}$. Image plane 12 in Fig. 3 has a finite thickness and ultrasound signals transmitted by beamforming ultrasound imaging probe 13 are detectable with reduced intensity at small out-of-plane displacements. In the same manner, beamforming ultrasound imaging probe 13 is sensitive to ultrasound reflections occurring at small out of plane displacements. These signals are used in the present invention to provide a qualitative indication of out-of-plane distance $D_{op}$ of ultrasound transducer 15.

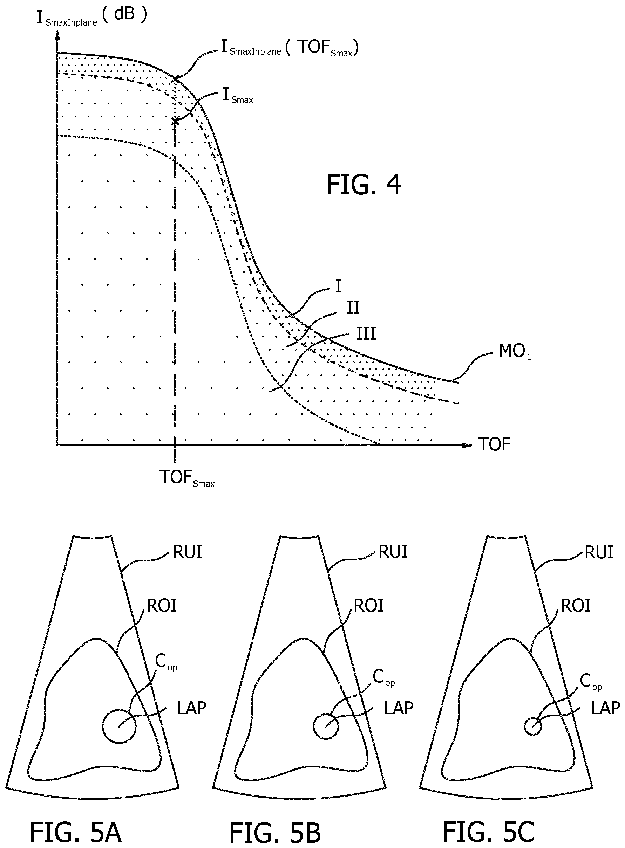

Thereto, Fig. 4 illustrates a model $MO_1$ describing an expected variation of in-plane maximum detected intensity, $I_{SmaxInplane}$ (dB) with time of flight, TOF. Model $MO_1$ is one model from a group of such models $MO_{1..n}$ that correspond to each of n different probe types, n being greater than or equal to two. The group of models may be stored as a database or library and thus selected therefrom. This may be provided by a memory. The memory may be included within the position determination unit or within another part of ultrasound imaging system 14; for example in a memory of imaging system processor ISP. Model $MO_1$ is indicated by the solid curve and illustrates that as the time of flight TOF, i.e. the depth into tissue, increases, the in-plane maximum detected intensity, $I_{SmaxInplane}$, of detected ultrasound signals initially decreases slowly, then more rapidly, and then more slowly again. The shape of the model is affected by attenuation of ultrasound signals and may be determined from theoretical calculations or empirical measurements of the in-plane maximum intensity obtained in tissue or corresponding matter. Model $MO_1$ depends only on time of flight and is invariant with in-plane angle $\theta_{IPA}$. It is noted that model $MO_1$ does not model the maximum detected intensity, $I_{SmaxInplane}$ as a function of out-of-plane distance. Consequently model $MO_1$ requires only a limited amount of, i.e. one-dimensional, calibration data. In contrast to a three-dimensional model, in-use the out-of-plane distance may be determined with model $MO_1$ with low latency due to the need to search in only one, i.e. time of flight, dimension. The modeled in-plane maximum detected intensity $I_{SmaxInplane}$ has been found to reliably represent different beamforming ultrasound imaging probes of the same type, which means that the same model may be used for beamforming ultrasound imaging probes of the same type. Moreover, there may be significant differences between probes of different types, and thus in some implementations, the type $T_{1..n}$ of the beamforming ultrasound imaging probe is used to assign a different model $MO_{1..n}$ to the corresponding probe type when computing the out of plane distance.

With reference to Fig. 3 and Fig. 4, in-use, computing out-of-plane distance $D_{op}$ comprises comparing the maximum detected intensity $I_{Smax}$ with model $MO_1$ for the corresponding probe type $T_1$. The out-of-plane distance $D_{op}$ may subsequently be indicated in reconstructed ultrasound image RUI. The out-of-plane distance may be indicated numerically for example, or as a size or color of an icon that varies accordance with $D_{op}$.

Comparing the maximum detected intensity $I_{Smax}$ with model $MO_1$ may for instance involve determining a difference or ratio between detected intensity $I_{Smax}$ and the in-plane maximum detected intensity, $I_{SmaxInplane}$ at the time of flight $TOF_{Smax}$ corresponding to the computed lateral position $LAP_{TOFSmax}$. In one exemplary implementation the maximum detected intensity $I_{Smax}$ at the computed lateral position $LAP_{TOFSmax, \theta IPA}$ of the ultrasound transducer may thus be scaled to the in-plane maximum detected intensity $I_{SmaxInplane}$, at the time of flight $TOF_{Smax}$ corresponding to the computed lateral position $LAP_{TOFSmax, \theta IPA}$. A qualitative indication of the out-of-plane distance may subsequently be indicated in reconstructed ultrasound image RUI. For example, an icon may be displayed that has a size that varies in accordance with:

$$Size = k_1 + k_2 \cdot \left(1 - \frac{I_{Smax}}{I_{SmaxInplane}}\right) \qquad \text{Equation 1}$$

and wherein $k_1$ and $k_2$ are constants and $k_1$ may include zero.

In another exemplary implementation the color of an icon may be configured to change based on the value of the maximum detected intensity $I_{Smax}$ in relation to $I_{SmaxInplane}$, at the time of flight $TOF_{Smax}$. For example, with reference to Fig. 4; zones I, II, and III, which represent predetermined ranges of $I_{Smax}$ or predetermine ranges of its ratio in relation to $I_{SmaxInplane}$, may define different colors of an icon displayed in reconstructed ultrasound image RUI, each color being applied to the icon when the maximum detected intensity $I_{Smax}$ lies in the respective range.

Thus, in summary, and with reference to Fig. 3 and Fig. 4, when imaging field $B_{1..k}$ comprises an image plane 12, computing the position $LAP_{TOFSmax, \theta IPA}$ of ultrasound transducer 15 respective imaging field $B_{1..k}$ may optionally further include determining an out-of-plane distance $D_{op}$ between ultrasound transducer and image plane based on the intensity Ismax and the time of flight $TOF_{Smax}$ of the maximum detected intensity ultrasound signal. Determining out-of-plane distance $D_{op}$ comprises selecting from a group of models $MO_{1..n}$ a model corresponding to the type $T_{1..n}$ of the beamforming ultrasound imaging probe 13 currently connected to ultrasound imaging system 14, the model describing an expected variation of in-plane maximum detected intensity $I_{SmaxInplane}$ with time of flight, and comparing the maximum detected intensity $I_{Smax}$ with the selected model, at the time of flight $TOF_{Smax}$ of the maximum detected intensity $I_{Smax}$ ultrasound signal. Indicating the position $LAPTOF_{Smax, \theta IPA}$ in the reconstructed ultrasound image RUI further comprises indicating the out-of-plane distance $D_{op}$ in reconstructed ultrasound image RUI.

By selecting the appropriate model from group of predetermined models $MO_{1..n}$, system 10 can operate reliably with different types of ultrasound imaging probe in out-of-plane positions.

In some exemplary implementations the out-of-plane distance $D_{op}$ may be indicated by means of a circular zone with a radius corresponding to the out-of-plane distance $D_{op}$. Thereto, Fig. 5A, Fig. 5B, Fig. 5C each illustrate a reconstructed ultrasound image RUI that includes region of interest ROI and an icon $C_{op}$ that is indicative of a circular zone with a radius corresponding to out-of-plane distance $D_{op}$. With reference to Fig. 5, indicating the out-of-plane distance $D_{op}$ may include providing icon $C_{op}$ at the computed lateral position $LAP_{TOFSmax, \theta IPA}$, the icon $C_{op}$ being indicative of a circular zone with a radius corresponding to the out-of-plane distance $D_{op}$. Fig. 5 also indicates region of interest ROI and within which the lateral position LAP of ultrasound transducer 15 has been determined. In Fig. 5A ultrasound transducer 15 is some distance from image plane 12 as indicated by the radius of circle $C_{op}$. Ultrasound transducer 15 is moved closer to image plane 12 throughout Fig. 5B and Fig. 5C, resulting in a corresponding reduction in the radius of circle $C_{op}$. Whilst a circle is indicated in Fig. 5, other icons than a complete circle and which are likewise indicative of a circular zone may be used in the same manner, including e.g. a circular arrangement of dots or dashes, a circular arrangement of radially-directed lines or arrows, the tips of which indicate a circular zone, and so forth. The use of an icon at the computed position with a circular zone indicative of the out-of-plane distance indicates intuitively to a user whether the interventional device is being advanced towards or away-from the image plane based on whether the circle grows or shrinks. This allows for improved guidance of the interventional device.

In some exemplary implementations the radius corresponding to out-of-plane distance $D_{op}$ is determined based on scaling the maximum detected intensity $I_{Smax}$ to the expected in-plane maximum detected intensity $I_{SmaxInplane}$, at the time of flight $TOF_{Smax}$ of the maximum detected intensity $I_{Smax}$ ultrasound signal. Thus, as described above with reference to Fig. 3, the radius of circle $C_{op}$ in Fig. 5 will change as ultrasound transducer 15 is moved towards and away from image plane 12.

In some exemplary implementations the icon $C_{op}$ has a perimeter and the appearance of the icon $C_{op}$ is configured to change based on a comparison of the maximum detected intensity $I_{Smax}$ with the expected in-plane maximum detected intensity $I_{SmaxInplane}$, at the time of flight $TOF_{Smax}$ of the maximum detected intensity $I_{Smax}$ ultrasound signal. The appearance of the icon $C_{op}$ may change by at least one of:

changing a color of the perimeter of the icon $C_{op}$;
a contrast of the perimeter of the icon $C_{op}$;
the perimeter of the icon $C_{op}$ with dots or dashes;
causing the perimeter of the icon $C_{op}$ to pulse over time;
if i) a ratio of the maximum detected intensity Ismax to the expected in-plane maximum detected intensity $I_{SmaxInPlane}$, at the time of flight $TOF_{Smax}$ of the maximum detected intensity $I_{Smax}$ ultrasound signal, or ii) the maximum detected intensity $I_{Smax}$, lies within a predetermined range. Other features of the icon may also be changed likewise, for example icon $C_{op}$ may take the form of a partially-transparent circular zone, under these conditions.

Changing the appearance of the perimeter has the effect of indicating to a user the position of the interventional device at predetermined positions respective the imaging plane. This feature allows the rapid indication to a user of the general position of the interventional device respective the imaging plane. For example, the with reference to zones I - III in Fig. 4, a color of the icon may be green when the maximum detected intensity or its ratio indicates a value close to the expected in-plane maximum detected intensity, i.e. in zone I, and red for values within an abutting range, i.e. in zone II, and white for positions outside this range, i.e. in zone III.

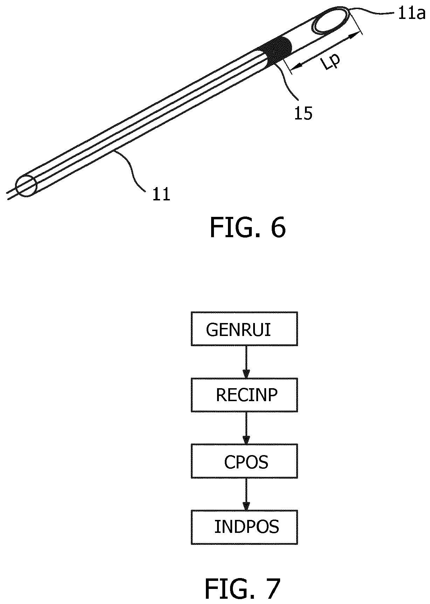

Fig. 6 illustrates an interventional device 11 that is suitable for use within system 10. Ultrasound transducer 15 maybe attached at a predetermined distance $L_p$ from a feature, i.e. distal end 11a of interventional device 11. Ultrasound transducer 15 may be attached to interventional device 11 by various means including using an adhesive. Electrical conductors that carry electrical signals from ultrasound transducer 11 to position determination unit PDU are also shown, although as mentioned above it is contemplated to alternatively use a wireless link to communicate the transducer signals with position determination unit PDU.

Ultrasound transducer 15 described above with reference to Fig. 1, Fig. 3 and Fig. 6 may be provided by a variety of piezoelectric materials. Both hard and soft piezoelectric materials are suitable. Micromachined Electromechanical Structures, i.e. MEMS devices such as Capacitive Machined Ultrasound Transducers, i.e. CMUT, devices are also suitable. When the ultrasound transducer is a detector, preferably it is formed from Polyvinylidene fluoride, otherwise known as PVDF whose mechanical properties and manufacturing processes lend themselves to attachment to curved surfaces such as medical needles. Alternative materials include a PVDF co-polymer such as polyvinylidene fluoride trifluoroethylene, a PVDF ter-polymer such as P(VDF-TrFE-CTFE). Preferably the ultrasound transducer is wrapped around an axis of the interventional device in order to provide sensing around 360 degrees of rotation about the axis although this need not always be the case.

In some exemplary implementations, position determination unit PDU may cause image reconstruction unit IRU to display said type $T_{1..n}$. This confirmation to a user may be provided in the reconstructed ultrasound image and provides reassurance that an automatically determined probe type has been correctly registered.

In some exemplary implementations, position determination unit PDU indicates the computed position $LAP_{TOFSmax, \theta IPA}$ only if the type $T_{1..n}$ corresponds to a type of probe in a group of two or more supported probe types. Consequently, whilst the ultrasound imaging system may operate with a variety of different probe types it is prevented that an incorrect position is displayed for probe types that are not supported by the position determination unit.

Fig. 7 illustrates various method steps of a method that may be used with system 10. With reference to Fig. 7, a method of determining a position of an interventional device 11 respective an imaging field $B_{1..k}$ corresponding to a type $T_{1..n}$ of a beamforming ultrasound imaging probe 13 currently connected to a beamforming ultrasound imaging system 14 and in which the position of the interventional device 11 is determined based on ultrasound signals transmitted between the beamforming ultrasound imaging probe 13 and an ultrasound transducer 15 attached to the interventional device 11 includes the steps of:

generating GENRUI a reconstructed ultrasound image RUI corresponding to the imaging field $B_{1..k}$ defined by the beamforming ultrasound imaging probe 13;

receiving input RECINP indicative of the type $T_{1..n}$ of the beamforming ultrasound imaging probe 13;

computing CPOS a position $LAP_{TOFSmax, \theta IPA}$ of the ultrasound transducer 15 respective the imaging field $B_{1..k}$ based on a time of flight $TOF_{Smax}$ of a maximum detected intensity $I_{Smax}$ ultrasound signal transmitted between the beamforming ultrasound imaging probe 13 and the ultrasound transducer 15, wherein computing the position $LAP_{TOFSmax, \theta IPA}$ comprises selecting from a group of beam sequences corresponding to a plurality of imaging probe types $T_{1..n}$ a beam sequence corresponding to the type $T_{1..n}$ of the beamforming ultrasound imaging probe 13 currently connected to the ultrasound imaging system 14, and assigning detected ultrasound signals to the selected beam sequence; and

indicating INDPOS the position $LAP_{TOFSmax, \theta IPA}$ in the reconstructed ultrasound image RUI.

It is to be noted that other implementations of the method may additionally incorporate one or more aspects described with respect to an implementation of the system.

The method steps illustrated in Fig. 7, optionally including other method steps described herein, may be stored on a computer program product as instructions that are executable by a processor. The computer program product may be provided by dedicated hardware, or hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD.

In this respect, a computer program product is also disclosed for use with system 10. The computer program product includes instructions which when executed on a processor of a system 10 for determining a position of an interventional device 11 respective an imaging field $B_{1..k}$ corresponding to a type $T_{1..n}$ of a beamforming ultrasound imaging probe 13 currently connected to a beamforming ultrasound imaging system 14 and in which the position of the interventional device 11 is determined based on ultrasound signals transmitted between the beamforming ultrasound imaging probe 13 and an ultrasound transducer 15 attached to the interventional device 11; cause the processor to carry out the aforementioned method steps.

In summary, a system is provided for determining a position of an interventional device respective an imaging field corresponding to a type of a beamforming ultrasound imaging probe currently connected to an ultrasound imaging system and in which the position of the interventional device is determined based on ultrasound signals transmitted between the beamforming ultrasound imaging probe and an ultrasound transducer attached to the interventional device. The system includes an image reconstruction unit and a position determination unit. The image reconstruction unit provides a reconstructed ultrasound image corresponding to the imaging field defined by the beamforming ultrasound imaging probe. The position determination unit receives input indicative of the type of the beamforming ultrasound imaging probe currently connected to the ultrasound imaging system. The position determination unit also computes a position of the ultrasound transducer respective the imaging field based on a time of flight of a maximum detected intensity ultrasound signal transmitted between the beamforming ultrasound imaging probe and the ultrasound transducer. Computing the position comprises selecting from a group of beam sequences corresponding to a plurality of imaging probe types a beam sequence corresponding to the type of the beamforming ultrasound imaging probe currently connected to the ultrasound imaging system and assigning detected ultrasound signals to the selected beam sequence. The position determination unit also indicates the position in the reconstructed ultrasound image.

Whilst the invention has been illustrated and described in detail in the drawings and foregoing description in relation to a medical needle, such illustrations and descriptions are to be considered illustrative or exemplary and not restrictive. Any reference signs in the claims should not be construed as limiting the scope of the invention. Moreover it is to be understood that the various examples, implementations and embodiments illustrated herein may be combined in order to provide various systems and methods for determining a position of an interventional device respective an image plane of a beamforming ultrasound imaging system.

**Claims**

1. System (10) for determining a position of an interventional device (11) respective an imaging field ($B_{1..k}$) corresponding to a type ($T_{1..n}$) of a beamforming ultrasound imaging probe (13) currently connected to an ultrasound imaging system (14) and in which the position of the interventional device (11) is determined based on ultrasound signals transmitted between the beamforming ultrasound imaging probe (13) and an ultrasound transducer (15) attached to the interventional device (11), the system (10) comprising:

   an image reconstruction unit (IRU) configured to provide a reconstructed ultrasound image (RUI) corresponding to the imaging field ($B_{1..k}$) defined by the beamforming ultrasound imaging probe (13); and
   a position determination unit (PDU) configured to:

   receive input indicative of the type ($T_{1..n}$) of the beamforming ultrasound imaging probe (13) currently connected to the ultrasound imaging system (14);
   compute a position ($LAP_{TOFsmax, \theta IPA}$) of the ultrasound transducer (15) respective the imaging field ($B_{1..k}$) based on a time of flight ($TOF_{Smax}$) of a maximum detected intensity ($I_{Smax}$) ultrasound signal transmitted between the beamforming ultrasound imaging probe (13) and the ultrasound transducer (15), wherein computing the position ($LAP_{TOFSmax, \theta IPA}$) comprises selecting from a group of beam sequences corresponding to a plurality of imaging probe types ($T_{1..n}$) a beam sequence corresponding to the type ($T_{1..n}$) of the beamforming ultrasound imaging probe (13) currently connected to the ultrasound imaging system (14) and assigning detected ultrasound signals to the selected beam sequence; and to
   indicate the position ($LAP_{TOFsmax, \theta IPA}$) in the reconstructed ultrasound image (RUI).

2. The system (10) according to claim 1 wherein the imaging field ($B_{1..k}$) comprises an image plane (12), and wherein computing the position ($LAP_{TOFSmax, \theta IPA}$) of the ultrasound transducer (15) respective the imaging field ($B_{1..k}$) further comprises determining an out-of-plane distance ($D_{op}$) between the ultrasound transducer (15) and the image plane (12) based on the intensity ($I_{Smax}$) and the time of flight ($TOF_{Smax}$) of the maximum detected intensity ultrasound signal;
   wherein determining the out-of-plane distance ($D_{op}$) comprises selecting from a group of models ($MO_{1..n}$) a model corresponding to the type ($T_{1..n}$) of the beamforming ultrasound imaging probe (13) currently connected to the ultrasound imaging system (14), the model describing an expected variation of in-plane maximum detected intensity ($I_{SmaxInplane}$) with time of flight, and comparing the maximum detected intensity ($I_{Smax}$) with the selected model, at the time of flight ($TOF_{Smax}$) of the maximum detected intensity ($I_{Smax}$) ultrasound signal; and
   wherein indicating the position ($LAPTOF_{Smax, \theta IPA}$) in the reconstructed ultrasound image (RUI) further comprises indicating the out-of-plane distance ($D_{op}$) in the reconstructed ultrasound image (RUI).

3. The system (10) according to claim 1 wherein the position determination unit (PDU) is configured to receive said input from the beamforming ultrasound imaging probe (13).

4. The system (10) according to any one of claims 1 - 3 wherein the position determination unit (PDU) is configured to cause the image reconstruction unit (IRU) to display said type ($T_{1..n}$).

5. The system (10) according to any one of claims 1 - 4 wherein the position determination unit is configured to indicate the position ($LAP_{TOFsmax, \theta IPA}$) only if the type ($T_{1..n}$) corresponds to a type of probe in a group of supported probe types.

6. The system (10) according to any previous claim further comprising the ultrasound imaging system (14), and wherein the image reconstruction unit (IRU) is included within the ultrasound imaging system (14).

7. The system (10) according to any previous claim further comprising a beamforming ultrasound imaging probe (13).

8. The system (10) according to any previous claim further comprising an interventional device (11) having an ultrasound transducer (15) attached thereto.

9. Method of determining a position of an interventional device (11) respective an imaging field ($B_{1..k}$) corresponding to a type ($T_{1..n}$) of a beamforming ultrasound imaging probe (13) currently connected to a beamforming ultrasound imaging system (14) and in which the position of the interventional device (11) is determined based on ultrasound signals transmitted between the beamforming ultrasound imaging probe (13) and an ultrasound transducer (15)

attached to the interventional device (11), the method comprising:

generating (GENRUI) a reconstructed ultrasound image (RUI) corresponding to the imaging field ($B_{1..k}$) defined by the beamforming ultrasound imaging probe (13);
receiving input (RECINP) indicative of the type ($T_{1..n}$) of the beamforming ultrasound imaging probe (13);
computing (CPOS) a position ($LAP_{TOFSmax,\,\theta IPA}$) of the ultrasound transducer (15) respective the imaging field ($B_{1..k}$) based on a time of flight ($TOF_{Smax}$) of a maximum detected intensity ($I_{Smax}$) ultrasound signal transmitted between the beamforming ultrasound imaging probe (13) and the ultrasound transducer (15), wherein computing the position ($LAP_{TOFSmax,\,\theta IPA}$) comprises selecting from a group of beam sequences corresponding to a plurality of imaging probe types ($T_{1..n}$) a beam sequence corresponding to the type ($T_{1..n}$) of the beamforming ultrasound imaging probe (13) currently connected to the ultrasound imaging system (14), and assigning detected ultrasound signals to the selected beam sequence; and
indicating (INDPOS) the position ($LAP_{TOFsmax,\,\theta IPA}$) in the reconstructed ultrasound image (RUI).

10. The method according to claim 9 wherein the imaging field ($B_{1..k}$) comprises an image plane (12), and wherein computing (CPOS) the position ($LAP_{TOFsmax,\,\theta IPA}$) of the ultrasound transducer (15) respective the imaging field ($B_{1..k}$) further comprises determining an out-of-plane distance ($D_{op}$) between the ultrasound transducer (15) and the image plane (12) based on the intensity ($I_{Smax}$) and the time of flight ($TOF_{Smax}$) of the maximum detected intensity ultrasound signal;

wherein determining the out-of-plane distance ($D_{op}$) comprises selecting from a group of models ($MO_{1..n}$) a model corresponding to the type ($T_{1..n}$) of the beamforming ultrasound imaging probe (13) currently connected to the ultrasound imaging system (14), the model describing an expected variation of in-plane maximum detected intensity ($I_{SmaxInplane}$) with time of flight, and comparing the maximum detected intensity ($I_{Smax}$) with the selected model, at the time of flight ($TOF_{Smax}$) of the maximum detected intensity ($I_{Smax}$) ultrasound signal; and

wherein indicating (INDPOS) the position ($LAPTOF_{Smax,\,\theta IPA}$) in the reconstructed ultrasound image (RUI) further comprises indicating the out-of-plane distance ($D_{op}$) in the reconstructed ultrasound image (RUI).

11. Computer program product comprising instructions which when executed on a processor of a system (10) for determining a position of an interventional device (11) respective an imaging field ($B_{1..k}$) corresponding to a type ($T_{1..n}$) of a beamforming ultrasound imaging probe (13) currently connected to a beamforming ultrasound imaging system (14) and in which the position of the interventional device (11) is determined based on ultrasound signals transmitted between the beamforming ultrasound imaging probe (13) and an ultrasound transducer (15) attached to the interventional device (11); cause the processor to carry out the method steps of claim 9 or claim 10.

FIG. 1

FIG. 2

FIG. 3

$I_{SmaxInplane}$ ( dB )

$I_{SmaxInplane}$ ( $TOF_{Smax}$ )

$I_{Smax}$

FIG. 4

I

II

III

$MO_1$

TOF

$TOF_{Smax}$

RUI

ROI

$C_{op}$

LAP

FIG. 5A

RUI

ROI

$C_{op}$

LAP

FIG. 5B

RUI

ROI

$C_{op}$

LAP

FIG. 5C

FIG. 6

GENRUI

RECINP

CPOS

INDPOS

FIG. 7

Europäisches
Patentamt

European
Patent Office

Office européen
des brevets

# EUROPEAN SEARCH REPORT

Application Number

EP 18 19 8791

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2018/060499 A1 (KONINKLIJKE PHILIPS NV [NL]) 5 April 2018 (2018-04-05) * page 7 - page 12 * ----- | 1-11 | INV. A61B8/08 |
| A | WO 2017/102369 A1 (KONINKLIJKE PHILIPS NV [NL]) 22 June 2017 (2017-06-22) * the whole document * ----- | 1-11 | |
| A,D | WO 2016/009350 A1 (KONINKL PHILIPS NV [NL]) 21 January 2016 (2016-01-21) * the whole document * ----- | 1-11 | |

TECHNICAL FIELDS
SEARCHED (IPC)

A61B

The present search report has been drawn up for all claims

| Place of search | Date of completion of the search | Examiner |
|---|---|---|
| Munich | 22 February 2019 | Koprinarov, Ivaylo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 632 331 A1**

## ANNEX TO THE EUROPEAN SEARCH REPORT
## ON EUROPEAN PATENT APPLICATION NO.

EP 18 19 8791

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2019

| Patent document cited in search report | | Publication date | Patent family member(s) | | Publication date |
|---|---|---|---|---|---|
| WO 2018060499 | A1 | 05-04-2018 | NONE | | |
| WO 2017102369 | A1 | 22-06-2017 | CN | 108369273 A | 03-08-2018 |
| | | | EP | 3391083 A1 | 24-10-2018 |
| | | | JP | 2018537227 A | 20-12-2018 |
| | | | US | 2018356493 A1 | 13-12-2018 |
| | | | WO | 2017102369 A1 | 22-06-2017 |
| WO 2016009350 | A1 | 21-01-2016 | CN | 106535774 A | 22-03-2017 |
| | | | EP | 3169244 A1 | 24-05-2017 |
| | | | JP | 2017520346 A | 27-07-2017 |
| | | | US | 2017202625 A1 | 20-07-2017 |
| | | | WO | 2016009350 A1 | 21-01-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011138698 A1
- WO 2015101949 A1
- WO 2016009350 A1
- WO 2018060499 A1